# EUROPEAN PATENT APPLICATION

(11) **EP 4 570 245 A1**
(43) Date of publication of application: **18.06.2025**
(21) Application number: 23216619.9
(22) Date of filing: 14.12.2023
(51) Int. Cl.: A61K 31/175, A61K 45/06, A61P 35/04, A61K 31/496

(54) **COMBINED AGENTS WITH SYNERGISTIC EFFECT AGAINST GLIOMAS**

(71) Applicant: Eberhard Karls Universität Tübingen Medizinische Fakultät, 72074 Tübingen (DE)
(72) Inventor: Tabatabai, Ghazaleh, 72076 Tübingen (DE); Walter, Bianca, 71083 Herrenberg (DE); Häußer, Lara Annina, 72076 Tübingen (DE); Kuhlburger, Laurence, 72070 Tübingen (DE); Merk, Daniel Josef Wolfgang, 71134 Aidlingen (DE)
(74) Representative: Witte, Weller & Partner Patentanwälte mbB

(57) **Abstract**

The present invention relates to a new pharmaceutical composition for the treatment and prophylaxis of a glioma comprising a first active agent combined with at least a second active agent, wherein the combination of said first and said second active agent results in a synergistic effect. The present invention also relates to a first active agent combined with at least a second active agent for use in the treatment and prophylaxis of a glioma wherein the combination of said first and said second active agent results in a synergistic effect. Finally, the invention relates to a method of treatment and prophylaxis of glioma in a subject in need, comprising the administration to the subject of said pharmaceutical composition or the combination of said first and said second active agent.

## Description

The present invention relates to a new pharmaceutical composition for the treatment and prophylaxis of a glioma comprising a first active agent combined with at least a second active agent, wherein the combination of said first and said second active agent results in a synergistic effect. The present invention also relates to a first active agent combined with at least a second active agent for use in the treatment and prophylaxis of a glioma wherein the combination of said first and said second active agent results in a synergistic effect. Finally, the invention relates to a method of treatment and prophylaxis of glioma in a subject in need, comprising the administration to the subject of said pharmaceutical composition or the combination of said first and said second active agent.

### FIELD OF THE INVENTION

The present invention relates to the field of drug development, more particular the field of active agents for the treatment and prophylaxis of a glioma.

Glioblastoma, previously known as glioblastoma multiforme (GBM), is the most aggressive and most common type of cancer that originates in the brain, and has very poor prognosis for survival. The cause of most cases of glioblastoma is not known. Uncommon risk factors include genetic disorders, such as neurofibromatosis and Li-Fraumeni syndrome, and previous radiation therapy. Glioblastomas represent 15% of all brain tumors. They are thought to arise from astrocytes.

Treatment usually involves surgery, after which chemotherapy and radiation therapy are used. The medication temozolomide is frequently used as part of chemotherapy. High-dose steroids may be used to help reduce swelling and decrease symptoms. Surgical removal (decompression) of the tumor is linked to increased survival, but only by some months.

Lomustine is often used in the progression of glioblastoma treatment. It is a chemotherapeutic alkylating nitrosourea compound closely related to semustine and is in the same family as streptozotocin. It is a highly lipid-soluble drug, thus it crosses the blood-brain barrier. Lomustine is a bifunctional alkylating agent, alkylates both DNA and RNA, has the ability to created interstrand cross-links (ICLs) in DNA. As with other nitrosoureas, it may also inhibit several key enzymatic processes by carbamoylation of amino acids in proteins. Lomustine is cell-cycle nonspecific.

Lomustine causes a variety of side effects including gastrointestinal, ocular, neurologic, and other disorders. Lomustine use is also linked to a variety of organ toxicities hepatotoxicity, nephrotoxicity, and pulmonary toxicity. Further, long term use of lomustine is linked to secondary malignancies including acute leukemia and myelodysplasia. Lomustine causes myelosupression in a delayed, dose-dependent, and cumulative fashion. Finally, fatal toxicity may occur with overdosage of lomustine.

Nevertheless, the anti-glioblastoma potential of lomustine is beyond doubt. Due to this potential and due to the availability and establishment of this drug, as well as the high development costs, it is desirable to provide a new and improved use of lomustine. In particular, such a new use should be provided to improve the efficacy of lomustine in the treatment and prophylaxis of gliomas, especially glioblastomas, and possibly reduce the observed side effects and toxicity.

Against this background, it is the object of the present invention to find an improved application of lomustine in the treatment and prophylaxis of gliomas and glioblastomas.

### SUMMARY OF THE INVENTION

This object is met by the provision of a pharmaceutical composition for use in the treatment and/or prophylaxis of glioma, said pharmaceutical composition comprising, in pharmaceutically/prophylactically effective amounts, a first active agent combined with at least a second active agent, wherein the combination of said first and said second active agent, when administered to a subject, results in a synergistic effect, wherein said first active agent is lomustine and said second active agent is an 'inhibitor of the Fanconi anemia pathway'.

According to the invention "lomustine" (CAS no.: 13010-47-4; INN; abbreviated as CCNU; original brand name CeeNU, now marketed as Gleostine) is a chemotherapeutic alkylating nitrosourea compound closely related to semustine and is in the same family as streptozotocin. It is a highly lipid-soluble drug, thus it crosses the blood-brain barrier. Lomustine is a bifunctional alkylating agent, alkylates both DNA and RNA, and has the ability to create interstrand cross-links (ICLs) in DNA. As with other nitrosoureas, it may also inhibit several key enzymatic processes by carbamoylation of amino acids in proteins. Lomustine is cell-cycle nonspecific.

The inventors used genome-wide CRISPR/Cas9 knockout screens under lomustine therapy to identify gene losses as functional vulnerabilities. These defects in the Fanconi anemia pathway were induced experimentally and a sensitization to lomustine therapy was detected. Using an established synergy assay, the inventors were able to verify that inhibitors of the Fanconi anemia pathway can potentiate the effect of lomustine. This can reduce the amount of lomustine used therapeutically or prophylactically and reduce side effects and toxicity.

According to the invention, the "Fanconi anemia pathway" or FA pathway is a mechanism of DNA repair for the resolution of interstrand crosslinks (ICLs). It is one of the most complex repair mechanisms of the cell, as it exhibits characteristics of homologous recombination, nucleotide excision repair and translesion synthesis. The FA pathway involves 22 recognized genes and specific mutations have been identified as the underlying defect in the majority of patients suffering from Fanconi anemia, a rare hereditary disease and form of anemia that can affect children and adults of any ethnic origin. It leads - via various pathways - to both reduced synthesis of red and white blood cells and excessive breakdown of these cells. The disease was named after the Swiss pediatrician Guido Fanconi.

"Inhibitors of the Fanconi anemia pathway" refer to a group of agents which can selectively and specifically target FA pathway members or genes/gene products, respectively, and reduce or even block its enzymatic activity.

In conclusion, the inventors were able to demonstrate that the combination of the first and at least second active agents results in a synergistic effect with respect to the treatment or prophylaxis of a glioma. "Synergy" according to the invention means that the at least two active agents reinforce each other's effect such that the total effect of the active agents is stronger than a summation (potentiation or superadditive effect).

Methods to determine synergistic effects of at least two active agents are well known in the art, including the Bliss independence model and the Zero interaction potency (ZIP) model; see, e.g., Ma and Motsinger-Reif (2019), Current methods for quantifying drug synergism, Proteom Bioinform. 1(2): 43-48, and Yadav et al. (2015), Searching for drug synergy in complex dose-response landscapes using an interaction potency model, Comput. Struct. Biotechnol. J. 13: 504-513.

According to the invention, "prophylaxis" means any measure intended to prevent a disease or pathologic condition, such as a glioma, from developing or progressing to a pathogenic form.

According to the invention, "treatment" or therapy refers to all measures aimed at positively influencing or improving a disease or pathologic condition, such as a glioma.

According to the invention "therapeutically/prophylactically effective amount" or, synonymously, "effective dose", refer to the amount or dose of the first and/or at least second active agent, that is sufficient to achieve the desired clinical outcome or improvement.

A "subject" according to the invention refers to any living being, including a vertebrate, a mammal, and a human being.

According to the invention the first and/or second active agents also include the respective pharmaceutically acceptable salts thereof, the solvates thereof and the solvates of the salts thereof. Solvates for the purposes of the invention refer to those forms of the active agents, which in the solid or liquid state form a complex by coordination with solvent molecules. Hydrates are a specific form of solvates in which the coordination takes place with water.

In the pharmaceutical composition the first and second active agents can be, in an embodiment of the invention, the only active agents. "At least" a second active agent means that, in alternative embodiments, a third, fourth, fifth etc. active agent is comprised by the pharmaceutical composition according to the invention.

It goes without saying that the pharmaceutical compositing according to the invention may comprise a pharmaceutically acceptable excipient or carrier.

"Pharmaceutically acceptable excipients or carriers" are well known to the skilled person. They allow a proper formulation of the compound according to the invention and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cotton-seed oil, safflower oil, sesame oil, olive oil, corn oil and soybean oil; glycols such as propylene glycol; esters, such as ethyl oleate and ethyl laurate; agar; buffering agents, such as magnesium hydroxide and aluminum hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Suitable pharmaceutical acceptable carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

"Comprising" according to the invention generally means encompassing all the specifically mentioned features as well as optional, additional unspecified ones. However, according to embodiments of the invention, "comprising" also includes "consisting of" which means that only those features are included which are explicitly specified.

The problem underlying the invention is hereby completely solved.

According to an embodiment of the invention said 'inhibitor of the Fanconi anemia pathway' is an agent inhibiting a target selected from the group consisting of: FANCA, FANCD2, FANCM, FANCB, FANCG, FANCE, FANCL, FANCI, BRIP1 (FANCJ), SLX4 (FANCP), UBE2T, FANCF, C17orf70, RFWD3, FAAP24, and BRCA1.

This measure has the advantage that a known member of the FA pathway is defined, which can then be inhibited in a targeted manner via specific and selective inhibitors. This potentiates the effect of lomustine. Inhibitors against these targets are therefore particularly well suited as second agents.

In yet another embodiment of the invention said 'inhibitor of the Fanconi anemia pathway' is PIP-199.

This measure has the advantage that an available, highly specific and selective inhibitor directed against the FA pathway member 'FANCM' is already in use. PIP-199 (CAS. no.: 622795-76-0) is a selective inhibitor of RMI (RecQ-mediated genome instability protein) core complex/MM2 interaction, with an IC50 of 36 µM). MM2 is the binding site of RMI complex on Fanconi anemia complementation group M protein (FANCM).

The inventors have carried out most of their experiments - by way of example - with the inhibitor PIP-199, but can conclude that other inhibitors of the Fanconi anemia pathway can also be used according to the invention in an advantageous manner, in particular those mentioned in the above embodiments.

In another embodiment of the invention said Fanconi anemia pathway is inhibited via RNA interference (RNAi).

This measure has the advantage that inhibitors are used that can be customized against any member of the FA pathway. RNA interference (RNAi) is a biological process in which RNA molecules are involved in sequence-specific suppression of gene expression by double-stranded RNA, through translational or transcriptional repression. Synthetic double-stranded RNA (dsRNA) introduced into cells can selectively and robustly induce suppression of specific genes of interest.

In yet another embodiment of the invention said 'inhibitor of the Fanconi anemia pathway' is selected from the group consisting of: short hairpin RNA (shRNA), microRNA (miRNA), and small interfering RNA (siRNA).

This measure is advantageous in that highly suitable molecules are already being used that make use of the RNA interference (RNAi) mechanism.

A short hairpin RNA or small hairpin RNA (shRNA/hairpin vector) is an artificial RNA molecule with a tight hairpin turn that can be used to silence target gene expression via RNAi. It typically consist of a stem of 19-29 base pairs (bp), a loop of at least 4 nucleotides (nt), and a dinucleotide overhang at the 3' end. Expression of shRNA in cells is typically accomplished by delivery of plasmids or through viral or bacterial vectors. shRNA is an advantageous mediator of RNAi in that it has a relatively low rate of degradation and turnover.

Small interfering RNA (siRNA), sometimes known as short interfering RNA or silencing RNA, is a class of double-stranded RNA at first non-coding RNA molecules, typically 20-24 (normally 21) base pairs in length, similar to miRNA, and operating within the RNAi pathway. It interferes with the expression of specific genes with complementary nucleotide sequences by degrading mRNA after transcription, preventing translation.

MicroRNA (miRNA) are small, single-stranded, non-coding RNA molecules containing 21 to 23 nucleotides. Mature miRNAs are structurally similar to siRNAs produced from exogenous dsRNA, but before reaching maturity, miRNAs must first undergo extensive post-transcriptional modification.

Each of the first and/or second active agent, in another embodiment, is present per dosage unit of the pharmaceutical composition at a concentration that, upon administration into a subject, results in a concentration in the glioma cells which is approx. ≤ 2000 µM, preferably about ≤ 1000 µM, further preferably ≤ 500 µM, further preferably ≤ 200 µM, further preferably ≤ 150 µM, further preferably ≤ 100 µM, further preferably ≤ 50 µM, further preferably ≤ 25 µM, further preferably ≤ 20 µM, further preferably ≤ 10 µM, further preferably ≤ 5 µM, further preferably ≤ 4 µM, further preferably ≤ 3 µM, further preferably ≤ 2.5 µM, further preferably ≤ 2 µM, and further preferably ≤ 1 µM.

This measure takes into account the findings of the inventors that setting the right therapeutic window by using the specified concentrations ensures on the one hand that the anti-glioma activity of the active agents is sufficient and on the other hand any cytotoxic effects are minimized.

In still another embodiment of the invention the glioma is a glioblastoma.

Glioblastoma, previously known as glioblastoma multiforme (GBM), is the most aggressive and most common type of cancer that originates in the brain, and has very poor prognosis for survival. There is no known method of preventing this cancer. Treatment usually involves surgery, after which chemotherapy and radiation therapy are used. However, the results achieved are often unsatisfactory and usually short-lived. Therefore, the invention provides a remedy here for the first time and provides an improved therapy and prophylaxis option.

In another embodiment of the invention the synergistic effect is determinable by the Bliss independence model and the Zero interaction potency (ZIP) model.

This measure has the advantage that the models are used which were al-so used by the inventors to find the combinations of active agents. They are therefore particularly suitable for determining the synergy. Both methods are well known to the skilled person; see, e.g., Ma and Motsinger-Reif *(loc. cit.)* and Yadav et al. (*loc. cit.*)*.*

Another subject-matter of the invention relates to a first active agent combined with at least a second active agent for use in the treatment and/or prophylaxis of a glioma, wherein the combination of said first and said second active agent, when administered to a subject, results in a synergistic therapeutic and/or prophylactic effect, wherein said first active agent is lomustine and said second active agent is an 'inhibitor of the Fanconi anemia pathway'.

The embodiments, properties, advantages, and features of the pharmaceutical composition according to the invention apply in a corresponding way to the combination for use according to the invention.

A still further subject-matter of the invention relates to a method of treatment and/or prophylaxis of glioma in a subject in need, comprising the administration to said subject of the pharmaceutical composition according to the invention or the first active agent combined with at least a second active agent for use according to the invention.

The embodiments, properties, advantages, and features of the pharmaceutical composition according to the invention apply in a corresponding way to the method according to the invention.

In an embodiment of the method according to the invention the first and the second active agent are administered simultaneously. In another embodiment of the method according to the invention the first and the second active agent are administered separately.

Due to this method of administration, their respective benefits can be selectively exploited and external circumstances of treatment can be accommodated. Simultaneous administration facilitates administration and promotes patient compliance or adherence to therapy. Separate administration can induce a priming effect via the agent administered first.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments and examples resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

### BRIEF DESCRIPTION OF THE FIGURES

- Fig. 1:: CRISPR screen analyses using a knockout library in LN18 (A, B) and T986 cells (C, D) to identify synthetic lethal interactors of lomustine (CCNU). A, C: Scatter plot of MAGeCK MLE results from comparing Brunello sgRNA distributions from DMSO or CCNU-treated cells to the plasmid library pool. B, D: Rankview plot illustrating MAGeCK MLE results from comparing Brunello sgRNA distributions from CCNU-treated cells to the corresponding DMSO control.
- Fig. 2:: Clonogenic survival assay results of shRNA knockdown cells of FANCA (A, C) or FANCD2 (B, D) upon CCNU treatment in comparison to a control shRNA targeting luciferase (shLuci). * p-value < 0.05
- Fig. 3:: Acute cytotoxicity synergy assay results of combination treatment of CCNU with the Fanconi anemia pathway inhibitor PIP-199 in MGMT promoter unmethylated cell lines LN 18 (A, D), T98G (B, E), and GS-2 (C, F). A-C: 3D synergy map. D-F: Heatmap.

### EXAMPLES

### 1. Material and methods

### Acute cytotoxicity assay

The inventors seeded either 5,000 or 10,000 cells, treated them on the following day, and incubated them for 72 h. Following this incubation, cell viability was assessed using CellTiterBlue reagent (Promega, Madison, Wl, US) in accordance with the manufacturer's instructions, utilizing a GloMax (Promega, Madison, Wl, US).

### Synergy assay

To assay drug combinations, the inventors treated the cells in 4x4 matrixes with lomustine and a second drug following the acute cytotoxicity assay protocol. The inventors analyzed the data for synergistic interactions with the R package syner-gyfinder to calculate zero interaction potential (ZIP) synergy scores for each tested combination in the 4x4 matrix and the average across the plate. Mean averages of two independent runs for each combination were then normalized over all combinations tested within a cell line. The highest average synergy value was set as 100% and a synergy value of 0 was set as 0%.

### Clonogenic survival assay

The inventors seeded the cells one day prior to treatment. The inventors then treated the cells with the specified concentrations in serum-free medium for 24 hours. Afterward, they changed the medium back to one containing FCS and incubated the cells for a period ranging from 14 to 21 days. The inventors stained the colonies using 0.5% w/v crystal violet and counted them. Measurements were normalized to the respective vehicle control wells.

### Primary glioblastoma cultures

The inventors used fresh residual material obtained from primary tumor tissue after resection at the Department of Neurosurgery, University Hospital Tübingen, to obtain primary glioblastoma cultures (approved by the ethics committee of the University Hospital Tübingen, vote number 225/2023 BO2). The inventors cut the tissue into small pieces, washed with Hanks Balanced Salt Solution (HBSS) (Gibco, Carlsbad, CA, US), and then digested using collagenase and dispase (Roche, Basel, CH). They used red blood lysis buffer (Sigma Aldrich, St. Louis, MO, US) to remove the remaining erythrocytes. For treatment, the inventors followed the acute cytotoxicity assay protocol. They treated the cells with lomustine and a second drug in monotherapy and combination. The inventors then used the Bliss Independence Criterion to evaluate synergism potential. They calculated a predicted value for additivity, the product of the two monotherapies, and compared it with the observed measurement of the combination treatment. Lower observed values compared to the prediction indicate towards synergy, higher towards antagonism.

### 2. Results

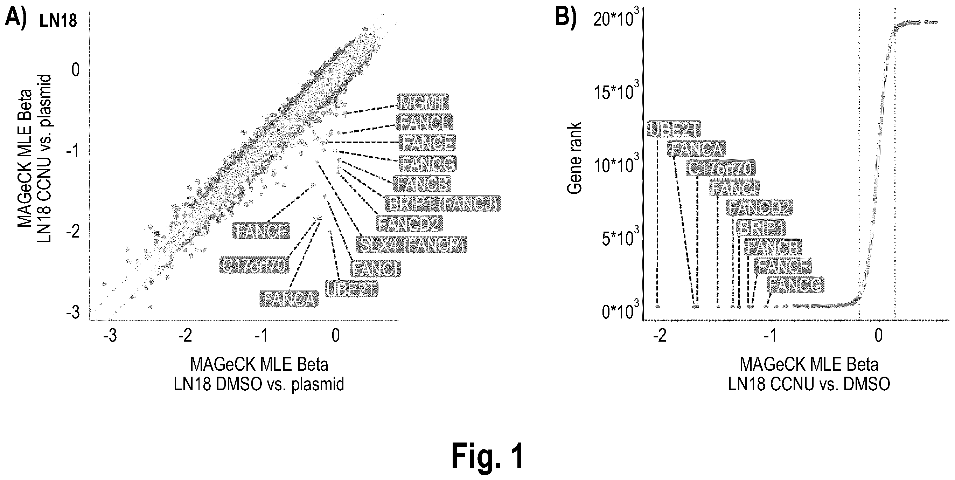

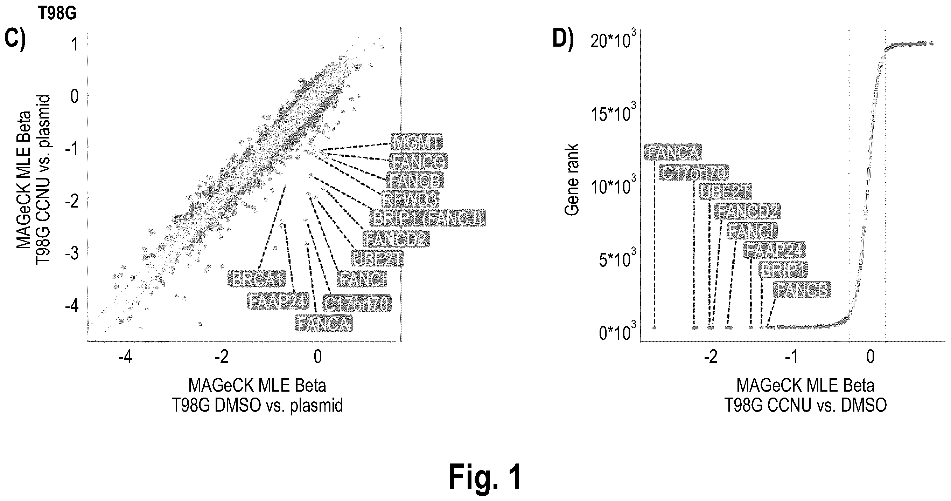

The alkylating agent lomustine (CCNU) is used in treatment of progressive glioblastoma. Patients with a methylated promoter of O-6-methylguanine-DNA methyltransferase (MGMT) can benefit from the treatment, but patients with an unmethylated promoter have a high need for improved treatment options. To identify synthetic lethal interactors of CCNU treatment in glioblastoma, the inventors performed genome-wide CRISPR/Cas9 knockout screens under treatment with CCNU or DMSO control. They used Brunello sgRNA library in MGMT promoter methylated/protein expressing glioma cell lines, LN18 and T98G, for the genome-wide CRISPR/Cas9 knockout screens (Figure 1).

Sequencing data was analyzed using MAGeCK MLE. Depleted genes in the CCNU treated sample which were not affected in the DMSO treated sample are synthetic lethal interactors of the treatment. Among those depleted genes the inventors identified 20 members of the Fanconi anemia pathway, including FANCA, FANCD2, FANCB, FANCG, FANCE, FANCL, FANCI, BRIP1 (FANCJ), SLX4 (FANCP), UBE2T, FANCF, C17orf70, RFWD3, FAAP24, and BRCA1.

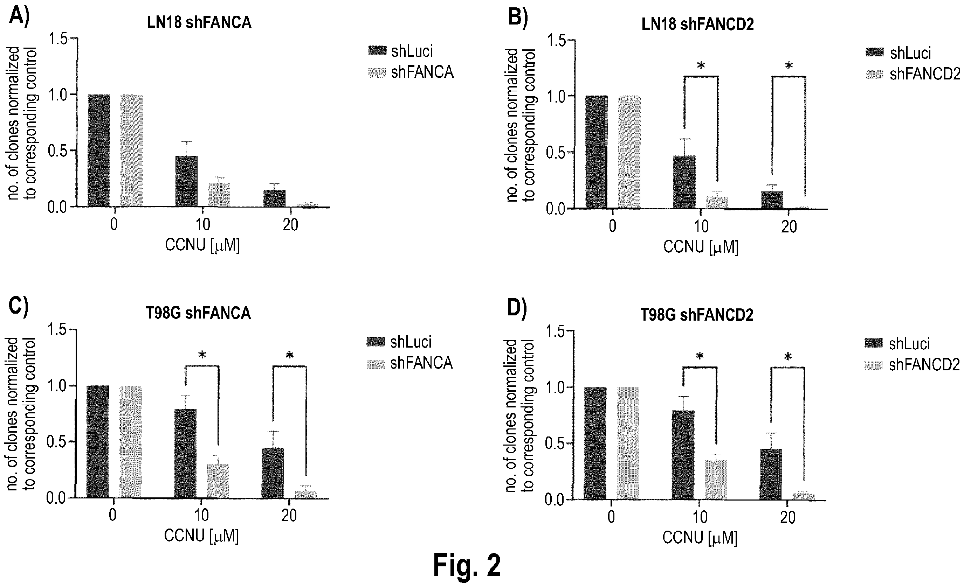

Based on the CRISPR/Cas9 screen result a loss of the Fanconi anemia DNA repair pathway should increase the effect of CCNU treatment. Therefore, the inventors induced an shRNA knockdown in LN18 and T98G glioma cell lines for FANCA or FANCD2. These knockdown cell lines were treated in clonogenic survival assays with CCNU in comparison to a control cell line transduced with a vector coding for an shRNA against Luciferase (Figure 2). FANCA and FANCD2 knockdown cell lines were more sensitive to CCNU treatment compared to the luciferase control.

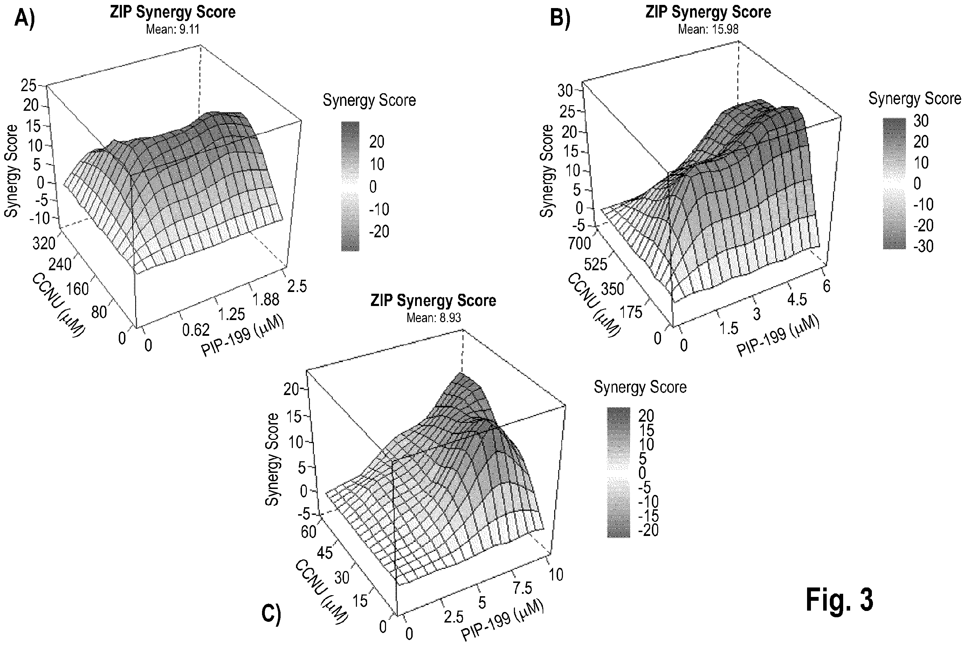

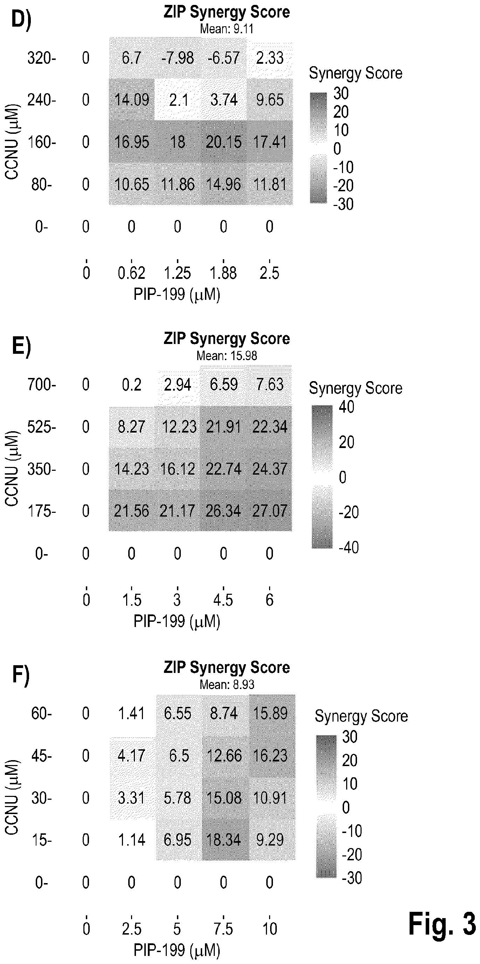

In addition, the inventors inhibited the Fanconi anemia pathway with the experimental drug PIP-199 and combined this treatment in parental cell lines with CCNU in acute cytotoxicity synergy assays (Figure 3).

The acute cytotoxicity assays were performed for 72 hours in LN18, T98G, GS-2, LN229, LNZ308, and GS-9 glioma cell lines in two independent runs. The combination of CCNU with PIP-199 was also analyzed in clonogenic survival assays in LN18, T98G, LN229, and LNZ308 with a synergistic effect upon combination treatment (data not shown).

For further validation the inventors also treated primary patient samples ex *vivo* with CCNU and PIP-199 mono- and combination treatments and detected a synergistic effect for the combination (data not shown).

### 3. Conclusion

With the present invention, the inventors provide for the first time a combination of active ingredients comprising lomustine and an 'inhibitor of the Fanconi anemia pathway' which, due to the observed clear synergistic effects of both components in the treatment and prophylaxis of gliomas, can be expected to lead to a significant therapeutic improvement over current treatment concepts. The inventors were able to demonstrate these effects in established experimental systems using representative example combinations.

## Claims

1. A pharmaceutical composition for use in the treatment and/or prophylaxis of glioma, said pharmaceutical composition comprising, in pharmaceutically/prophylactically effective amounts, a first active agent combined with at least a second active agent, wherein the combination of said first and said second active agent, when administered to a subject, results in a synergistic effect, wherein said first active agent is lomustine and said second active agent is an 'inhibitor of the Fanconi anemia pathway'.

2. The pharmaceutical composition of use of claim 1, wherein said 'inhibitor of the Fanconi anemia pathway' is an agent inhibiting a target selected from the group consisting of: FANCA, FANCD2, FANCM, FANCB, FANCG, FANCE, FANCL, FANCI, BRIP1 (FANCJ), SLX4 (FANCP), UBE2T, FANCF, C17orf70, RFWD3, FAAP24, and BRCA1.

3. The pharmaceutical composition for use of claim 1 or 2, wherein said 'inhibitor of the Fanconi anemia pathway' is PIP-199.

4. The pharmaceutical composition for use of any of the preceding claims, wherein said Fanconi anemia pathway is inhibited via RNA interference (RNAi).

5. The pharmaceutical composition for use of claim 4, wherein said 'inhibitor of the Fanconi anemia pathway' is selected from the group consisting of: short hairpin RNA (shRNA), microRNA (miRNA), and small interfering RNA (siRNA).

6. The pharmaceutical composition of any of the preceding claims, wherein the glioma is a glioblastoma.

7. The pharmaceutical composition of any of the preceding claims, wherein the synergistic effect is determinable by Bliss independence model and the Zero interaction potency (ZIP) model.

8. A first active agent combined with at least a second active agent for use in the treatment and/or prophylaxis of a glioma, wherein the combination of said first and said second active agent, when administered to a subject, results in a synergistic therapeutic and/or prophylactic effect, wherein said first active agent is lomustine and said second active agent is an 'inhibitor of the Fanconi anemia pathway'.

9. The first active agent combined with at least a second active agent for use of claim 8, wherein the said 'inhibitor of the Fanconi anemia pathway' is an agent inhibiting a target selected from the group consisting of: FANCA, FANCD2, FANCM, FANCB, FANCG, FANCE, FANCL, FANCI, BRIP1 (FANCJ), SLX4 (FANCP), UBE2T, FANCF, C17orf70, RFWD3, FAAP24, and BRCA1.

10. The first active agent combined with at least a second active agent for use of claim 8 or 9, wherein the said 'inhibitor of the Fanconi anemia pathway' is PIP-199.

11. The first active agent combined with at least a second active agent for use of any of claims 8-10, wherein said Fanconi anemia pathway is inhibited via RNA interference (RNAi).

12. The first active agent combined with at least a second active agent for use of claim 11, wherein said 'inhibitor of the Fanconi anemia pathway' is selected from the group consisting of: short hairpin RNA (shRNA), microRNA (miRNA), and small interfering RNA (siRNA).

13. A method of treatment and/or prophylaxis of glioma in a subject in need, comprising the administration to said subject of the pharmaceutical composition of any of claims 1-7 or the first active agent combined with at least a second active agent for use of any of claims 8-12.

14. The method of claim 13, wherein the first and the second active agent are administered simultaneously.

15. The method of claim 13, wherein the first and the second active agent are administered separately.
